# EUROPEAN PATENT SPECIFICATION

(11) **EP 4 005 625 B1**
(45) Date of publication and mention of the grant of the patent: **16.04.2025**
(21) Application number: 20846360.4
(22) Date of filing: 27.07.2020
(51) Int. Cl.: A61M 37/00, A61B 17/22, A61M 25/00, A61N 7/00

(54) **VORTEX CATHETER THROMBOLYTIC SYSTEM**
THROMBOLYTISCHES WIRBELKATHETERSYSTEM
SYSTÈME THROMBOLYTIQUE DE CATHÉTER À VORTEX

(30) Priority: 30.07.2019 US 201916525648
(43) Date of publication of application: 01.06.2022
(73) Proprietor: National Tsing Hua University, Hsinchu City 30013 (TW)
(72) Inventor: YEH, Chih-Kuang, Hsinchu City, Taiwan 30013 (TW); LO, Wei-Chen, Hsinchu City, Taiwan 30013 (TW)
(74) Representative: Kuhnen & Wacker Patent- und Rechtsanwaltsbüro PartG mbB
(86) International application number: PCT/CN2020/104744
(87) International publication number: WO 2021/018074

(56) References cited:
- EP-A1- 2 170 181
- CN-A- 105 025 977
- CN-A- 108 187 183
- CN-A- 108 888 313
- CN-A- 109 432 586
- CN-U- 205 548 629
- US-A1- 2014 276 367
- US-A1- 2017 281 130
- US-A1- 2018 071 505
- US-A1- 2018 071 505

## Description

### BACKGROUND

### Technical Field

The present invention relates to a vortex catheter device, and more specifically relates to a vortex catheter thrombolytic system.

### Related Art

Thrombolytic therapy for general vascular thrombosis mainly relies on drugs, including anticoagulants and thrombolytic agents. A thrombolytic agent, such as a tissue plasminogen activator (Tpa), is taken as an example herein. The Tpa is an anticoagulant that was first approved by the US Food and Drug Administration (FDA) to treat thrombusinduced stroke. However, the Tpa also has serious defects. Thrombolytic drugs have the risk of causing bleeding, the Tpa must be used within hours of the onset of stroke symptoms, the drug effect in the body can not be long-lasting and may cause long-lasting bleeding, and large blood clots can not be dissolved usually. If high-dose thrombolytic drugs are used, it is easy to cause massive bleeding in the body. In addition, patent document US 2018/071505 A1 discloses an ultrasonic device for transversely manipulating drug delivery carriers and method using the same; patent document US 2014/276367 A1 discloses a method and apparatus for drug delivery to a target site; patent document EP 2 170 181 A1 discloses a method and apparatus for treatment of intracranial hemorrhages; and patent document US 2017/281130 A1 discloses a method and apparatus for accelerated disintegration of blood clot.

Generally, ultrasonic waves refer to generally-focused or non-focused ultrasonic waves. In the technical field of ultrasonic emission, it is a general prior art to generate an ultrasonic focusing effect by phase modulation. In order to reduce the dose of thrombolytic drugs and the risk of bleeding and consider the thrombolytic effect, an interventional catheter is used for directly delivering the thrombolytic agents to the thrombus, and general ultrasonic waves are used for strengthening the drugs to ablate the thrombus so as to strengthen the thrombolytic effect. However, general phase modulation does not have the thrombolytic technology for generating a vortex acoustic field by phase modulation of the present invention.

Based on the above, the present invention adopts phase modulation to generate a vortex acoustic field, and the general phase modulation and the phase modulation of the present invention for generating the vortex acoustic field are two completely different technologies. The probe for transmitting the vortex acoustic field is provided with a piezoelectric patch, the piezoelectric patch comprises a plurality of channels, and the phase difference generated between every two channels is used for generating a vortex of an acoustic channel by the ultrasonic transducer. In brief, although the two technologies are based on phase modulation, the objectives are different.

### SUMMARY

The problem to be solved by the present invention is that the technology for generating a vortex acoustic field through phase modulation makes the present invention superior to the general ultrasonic combined thrombolytic drug technology to improve the efficiency in a thrombolytic method.

The invention is defined by independent claim 1. Further developments are defined by the dependent claims.

In order to further understand the objectives, effects, features and structures of the present invention, exemplary embodiments in conjunction with accompanying drawings are illustrated below.

### BRIEF DESCRIPTION OF THE DRAWINGS

FIG. 1 is a schematic diagram of a vortex catheter thrombolytic system with an ultrasonic transducer;
FIG. 2 is a schematic diagram of a vortex catheter thrombolytic system;
FIG. 3 is a schematic diagram of a vortex catheter thrombolytic system;
FIG. 4 is a schematic diagram of a vortex catheter thrombolytic system according to an embodiment of the present invention;
FIG. 5 is a flow diagram of a thrombolytic method in whichan embodiment of the present invention may be employed;
FIG. 6 is a diagram showing a dissolution rate of a thrombolytic experiment under three experiment conditions according to an embodiment of the present disclosure.

### DETAILED DESCRIPTION

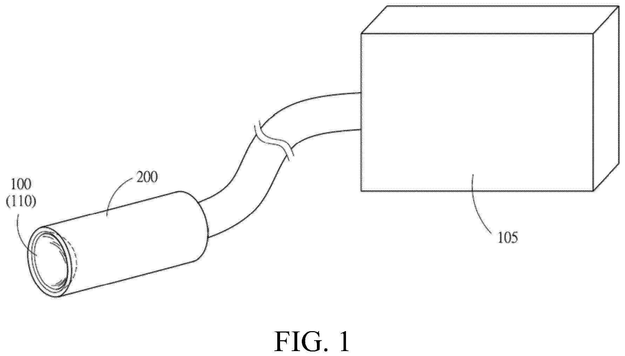

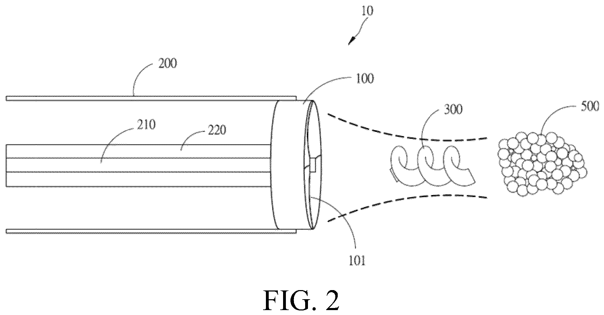

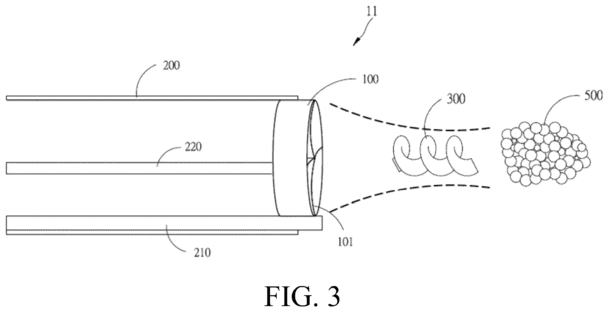

FIG. 1 is a schematic diagram of a vortex catheter thrombolytic system with an ultrasonic transducer, FIG. 2 is a schematic diagram of a vortex catheter thrombolytic system, and FIG. 3 is a schematic diagram of a vortex catheter thrombolytic system. FIG. 1 to FIG.3 illustrate aspects of the disclosure but are not according to the claimed embodiment. Referring to FIG. 1 and FIG. 2, in an embodiment of the present disclosure, a vortex catheter thrombolytic system 10 comprises an ultrasonic transducer 105; and a probe 100 for transmitting a vortex acoustic field as well as a catheter 200, wherein the catheter 200 is arranged in the probe 100 for transmitting the vortex acoustic field, is connected to the ultrasonic transducer 105 and is provided with a first inner channel 210 and a second inner channel 220, the first inner channel 210 is used for delivering drugs, the second inner channel 220 is used for vortex driving, the probe 100 for transmitting the vortex acoustic field is provided with a piezoelectric patch 101, the piezoelectric patch 101 comprises a plurality of channels, and the phase difference generated between every two channels is used for generating a vortex 300 of an acoustic channel by the ultrasonic transducer 105.

Referring to FIG. 1 and FIG. 3, in an embodiment of the present disclosure, a vortex catheter thrombolytic system 11 comprises an ultrasonic transducer 105; and a probe 100 for transmitting a vortex acoustic field as well as a catheter 200, wherein the catheter 200 is arranged in the probe 100 for transmitting the vortex acoustic field, is connected to the ultrasonic transducer 105 and is provided with a first inner channel 210 and a second inner channel 220, the first inner channel 210 is used for delivering drugs, the second inner channel 220 is used for vortex driving, the probe 100 for transmitting the vortex acoustic field is provided with a piezoelectric patch 101, the piezoelectric patch 101 comprises a plurality of channels, and the phase difference generated between every two channels is used for generating a vortex 300 of an acoustic channel by the ultrasonic transducer 105.

Specifically, the ultrasonic transducer 105 can be a pulse generator. More specifically, the ultrasonic transducer 105 can be, but not limited to, a pulse generator based on a field programmable gate array (FPGA). Furthermore, a driving signal transmitted by the ultrasonic transducer 105 can be a square wave signal or a sine wave signal. Although not shown in the figures, an amplifier can be arranged on the ultrasonic transducer 105 to amplify the driving signal.

In an embodiment of the present disclosure, the piezoelectric patch 101 is made from a lead zirconate titanate material. Furthermore, the piezoelectric patch 101 and a filling and sealing casing are filled with epoxy resin, but the present invention is not limited thereto.

In an embodiment of the present invention, the probe 100 for transmitting the vortex acoustic field can be connected with the catheter 200 in a magnetic adsorption mode, a buckling mode or a gluing mode.

In an embodiment of the present invention, the probe 100 for transmitting the vortex acoustic field comprises at least one piezoelectric patch 101 or comprises multiple array elements (at least four array elements) of piezoelectric patches 101, the piezoelectric patch 101 has a curved shape and is cut into four adjacent channels, and the phase difference generated between every two adjacent channels is used for generating an acoustic vortex.

In an embodiment of the present invention, the curvature radius of the piezoelectric patch ranges from 1 mm to 300 mm.

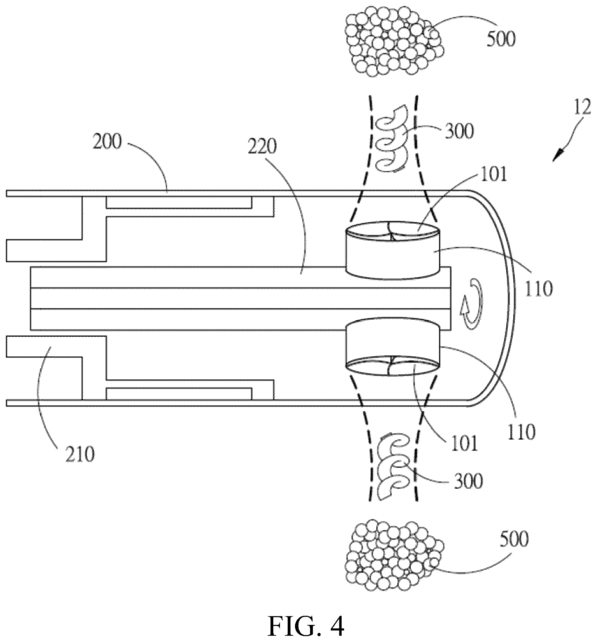

FIG. 4 is a schematic diagram of a vortex catheter thrombolytic system according to an embodiment of the present invention. Referring to FIG. 1 and FIG. 4, in an embodiment of the present invention, a vortex catheter thrombolytic system 12 comprises an ultrasonic transducer 105; and a radial probe 110 for transmitting a vortex acoustic field as well as a catheter 200, wherein the catheter 200 is arranged in the radial probe 110 for transmitting the vortex acoustic field, is connected to the ultrasonic transducer 105 and is provided with a first inner channel 210 and a second inner channel 220, the first inner channel 210 is used for delivering drugs, the second inner channel 220 is used for vortex driving, the radial probe 110 for transmitting the vortex acoustic field can perform vortex motion at two sides so as to increase the vortex driving to the radial direction of blood vessels, the radial probe 110 for transmitting the vortex acoustic field is provided with a piezoelectric patch 101, the piezoelectric patch 101 comprises a plurality of channels, and the phase difference generated between every two channels is used for generating a vortex 300 of an acoustic channel by the ultrasonic transducer 105.

In an embodiment of the present invention, the radial probe 110 for transmitting the vortex acoustic field comprises at least one piezoelectric patch 101 or comprises multiple array elements (at least four array elements) of piezoelectric patches 101, the piezoelectric patch 101 has a curved shape and is cut into four adjacent channels, and the phase difference generated between every two adjacent channels is used for generating an acoustic vortex.

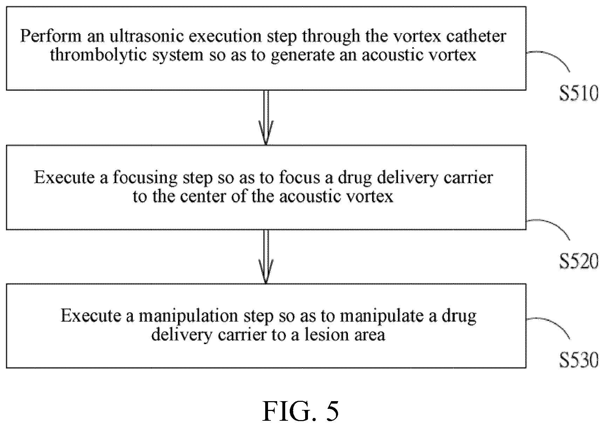

FIG. 5 is a flow diagram of a thrombolytic method in which an embodiment of the invention may be employed. Referring to FIG. 5, a thrombolytic method comprises: performing an ultrasonic execution step through the vortex catheter thrombolytic systems 10, 11 and 12 so as to generate an acoustic vortex; executing a focusing step so as to focus a drug delivery carrier to the center of the acoustic vortex; and executing a manipulation step so as to manipulate the drug delivery carrier to a lesion area.

The ultrasonic execution step is executed by a pulse generator having a duty cycle of 30% or higher.

The parameters in the ultrasonic execution step are as follows: the frequency is 0.5-20 MHz, and the acoustic pressure ranges from 0.1 MPa to 2 MPa.

Referring to FIG. 5, in S510, the ultrasonic execution step is performed through the vortex catheter thrombolytic system so as to generate an acoustic vortex.

Referring to FIG. 5, in S520, the focusing step is executed so as to focus a drug delivery carrier to the center of the acoustic vortex.

Referring to FIG. 5, in S530, the manipulation step is executed so as to manipulate a drug delivery carrier to a lesion area.

Thrombus is a blood clot formed in blood vessels and acts in a blood circulation system to obstruct or block blood flow. When the blood vessels are damaged, in order to avoid blood loss or further damage to the blood vessels caused by blood flow impact, platelets and fibrin in the blood will aggregate to form the blood clot for repairing. However, if the blood clot falls off, it may cause thrombosis to cause embolism.

### Experiment method

Preparation of thrombus 500 and simulation of blood flow:
The blood of the human body is divided into plasma and erythrocyte. The plasma accounts for about 55% of the total blood volume, and the erythrocyte accounts for about 45% of the total blood volume. The thrombus can also be prepared by using whole blood. The thrombus in this experiment is prepared from the whole blood of the human body. There may be some slight differences between individuals, and the concentration of calcium chloride can be finely adjusted.

A thrombus preparation formula in the experiment does not require a special ratio and basically comprises 55% of plasma and 45% of erythrocyte. The experiment can also be performed according to the data provided by the medical corporate body Taiwan Blood Services Foundation.

Step 1: plasma, erythrocyte and thrombin 20U were placed in a constanttemperature water tank and heated to 37°C.

Step 2: the plasma, the erythrocyte, the thrombin 20U and calcium chloride (CaCl₂) were sequentially added in proportion.

Step 3: after mixing, a syringe was fixed by a floating pad and placed at the water temperature of 37°C for 1 h to form a thrombus to be pushed out by the syringe.

Step 4: an Actilyse solution has a function of dissolving the thrombus 500, a solution containing Actilyse (scientific name: Alteplase) was used as a thrombolytic drug (Tpa) in this experiment, the prepared thrombus was added to the thrombolytic drug prepared from the Actilyse solution, then, a solidified state was changed into a flow state, and a peristaltic pump was used for generating a flow environment so as to simulate the blood flow.

**Table 1 Thrombus preparation and blood flow simulation experiment material composition**

| Experiment material | Parameter |
|---|---|
| Ultrasonic wave | Frequency: 0.5-20 MHz |
| | Acoustic pressure: 1.6 MPa |
| | Duty cycle: 50% |
| Thrombolytic drug | Actilyse |
| Thrombus preparation material | Erythrocyte |
| | Plasma |
| | Thrombin |
| | CaCl₂ |

Thrombolytic experiment method:
At the simulated body temperature of 37°C, three groups of experiment conditions (one control group and two experiment groups) were provided: a thrombolytic drug (Tpa), a general ultrasonic wave combined thrombolytic drug (Tpa), and a vortex acoustic field combined thrombolytic drug (Tpa). In order to compare the difference between the control group and the experiment groups, the experiment conditions were fixed and dissolved for 1 h for comparison, and the thrombolytic effects of all groups were obtained by comparing the residual dose of thrombus dissolution.

### Control group

Thrombolytic drug (Tpa): the thrombolytic drug was added to the thrombus 500, and the residual dose of thrombus dissolution was calculated 1 h later.

### Experiment group 1

General ultrasonic wave combined thrombolytic drug (Tpa): general ultrasonic waves were applied to the thrombus 500 under the conditions that the pressure was 1.6 MPa and the duty cycle was 50%, and the residual dose of thrombus dissolution was calculated 1 h later.

### Experiment group 2

Vortex acoustic field combined thrombolytic drug (Tpa): a vortex acoustic field was applied to the thrombus 500 under the conditions that the pressure was 1.6 MPa and the duty cycle was 50%, and the residual dose of thrombus dissolution was calculated 1 h later.

### Thrombolytic experiment result

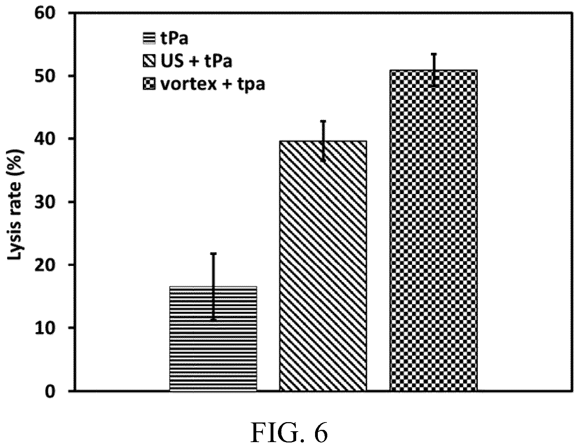

FIG. 6 shows a dissolution rate of a thrombolytic experiment under three experiment conditions.

The dissolution rate of the thrombolytic experiment under three experiment conditions: experiment group 2: the dissolution rate of the vortex acoustic field combined thrombolytic drug (Tpa) was 51%; experiment group 1: the dissolution rate of the general ultrasonic wave combined thrombolytic drug (Tpa) was 40%; and control group: the dissolution rate of the thrombolytic drug (Tpa) was 17%. The results of many experiments show that the thrombus dissolution rate of the experiment group 2 was increased by more than 10% compared with the experiment group 1, and the difference was significant; and the thrombus dissolution rate of the experiment group 2 was increased by more than 34% compared with the control group, and the difference was also significant.

The results of many experiments show that the thrombolytic effect of the vortex acoustic field combined thrombolytic drug (Tpa) was better than that of the general ultrasonic wave combined thrombolytic drug (Tpa), more than 10% of the thrombus dissolution rate was increased, and the difference was significant; and the thrombolytic effect of the vortex acoustic field combined thrombolytic drug (Tpa) was better than that of the single thrombolytic drug (Tpa), more than 34% of the thrombus dissolution rate was increased, and the difference was also significant. The experiments prove that the dissolution rate of the vortex acoustic field combined thrombolytic drug (Tpa) applied to thrombus dissolution was better than that of other prior arts.

The experiments prove that the dissolution rate of the vortex acoustic field combined thrombolytic drug (Tpa) applied to the thrombolytic experiment was better than that of other prior arts.

The probe for transmitting the vortex acoustic field or the radial probe for transmitting the vortex acoustic field of the present invention can enhance the vortex driving effect and achieve the objective of quick thrombus dissolution.

Therefore, the present invention has excellent advancement and practicability in similar products.

The above embodiments are merely exemplary embodiments of the present invention, and other structural changes made by the specifications and claims of the present invention are intended to be included in the scope of the claims of the present invention, as long as these changes fall within the scope of protection which is defined solely by the claims.

## Claims

1. A vortex catheter thrombolytic system (12), comprising an ultrasonic transducer (105), a radial probe (110) for transmitting a vortex acoustic field, and a catheter (200), wherein:
the catheter (200) is connected to the ultrasonic transducer (105) and is provided with a first inner channel (210) and a second inner channel (220), the first inner channel (210) is configured for delivering drugs, the second inner channel (220) is provided with a piezoelectric patch (101) on the radial probe (110) configured for vortex driving, and the piezoelectric patch (101) comprises four channels, **characterized in that**:
the four channels are disposed in a radial direction of the catheter (200), wherein a phase difference between any two adjacent channels of the piezoelectric patch (101) is configured for generating an acoustic vortex (300) in the radial direction of the catheter (200) by the ultrasonic transducer (105);
the piezoelectric patch (101) is enveloped in the catheter (200) and is not exposed out of the catheter (200); and
a delivering exit where the drugs are delivered out is adjacent to the acoustic vortex (300) generated by the piezoelectric patch (101), whereby the drugs are delivered out and focused on a center of the acoustic vortex (300) in the radial direction of the catheter (200).

2. The vortex catheter thrombolytic system (12) according to claim 1, wherein the probe (110) for transmitting the vortex acoustic field is connected with the catheter (200) in a magnetic adsorption mode, a buckling mode or a gluing mode.

3. The vortex catheter thrombolytic system (12) according to claim 1, wherein the probe (110) for transmitting the vortex acoustic field comprises at least one piezoelectric patch (101) or comprises multiple array elements (at least four array elements) of piezoelectric patches (101).

4. The vortex catheter thrombolytic system (12) according to claim 3, wherein the curvature radius of the piezoelectric patch (101) ranges from 1 mm to 300 mm.

5. The vortex catheter thrombolytic system (12) according to claim 1, wherein:
the vortex catheter thrombolytic system (12) further comprises another radial probe (110) for transmitting a vortex acoustic field;
the second inner channel (220) is further provided with another piezoelectric patch, the two piezoelectric patches (101) on the two radial probes (110) are respectively used for vortex driving, the two radial probes (110) for transmitting the vortex acoustic field is configured to perform vortex motion at two sides, one of piezoelectric patches (101) comprises four channels disposed in a radial direction of the catheter (200), the other one of piezoelectric patches (101) comprises four channels disposed in another radial direction of the catheter (200), wherein a phase difference between any two adjacent channels of each piezoelectric patch (101) is used for generating an acoustic vortex (300) in the corresponding radial direction of the catheter (200) by the ultrasonic transducer (105);
each piezoelectric patch (101) is enveloped in the catheter (200) and is not exposed out of the catheter (200);
each delivering exit where the drugs are delivered out is adjacent to the acoustic vortex (300) generated by the piezoelectric patch (101), whereby the drugs are delivered out and focused on a center of the acoustic vortex (300) in the corresponding radial direction of the catheter (200).

## Patentansprüche

1. Vortex-Katheter-Thrombolyse-System (12), umfassend einen Ultraschallwandler (105), eine radiale Sonde (110) zur Übertragung eines akustischen Vortex-Feldes und einen Katheter (200), wobei:
der Katheter (200) mit dem Ultraschallwandler (105) verbunden ist und mit einem ersten inneren Kanal (210) und einem zweiten inneren Kanal (220) versehen ist, wobei der erste innere Kanal (210) zur Abgabe von Medikamenten konfiguriert ist, der zweite innere Kanal (220) mit einer piezoelektrischen Stelle (101) an der radialen Sonde (110) versehen ist, die zur Vortex-Erzeugung konfiguriert ist, und die piezoelektrische Stelle (101) vier Kanäle umfasst, **dadurch gekennzeichnet, dass**:
die vier Kanäle in radialer Richtung des Katheters (200) angeordnet sind, wobei eine Phasenverschiebung zwischen zwei beliebigen benachbarten Kanälen der piezoelektrischen Stelle (101) zur Erzeugung eines akustischen Vortex (300) in radialer Richtung des Katheters (200) durch den Ultraschallwandler (105) konfiguriert ist;
die piezoelektrische Stelle (101) im Katheter (200) eingeschlossen ist und nicht aus dem Katheter (200) herausragt; und
ein Abgabeausgang, an dem die Medikamente abgegeben werden, in der Nähe des durch die piezoelektrische Stelle (101) erzeugten akustischen Vortex (300) ist, wodurch die Medikamente abgegeben und auf ein Zentrum des akustischen Vortex (300) in radialer Richtung des Katheters (200) fokussiert werden.

2. Vortex-Katheter-Thrombolyse-System (12) nach Anspruch 1, wobei die Sonde (110) zur Übertragung des akustischen Vortex-Feldes mit dem Katheter (200) durch magnetische Adsorption, durch Verriegelung oder durch Kleben verbunden ist.

3. Vortex-Katheter-Thrombolyse-System (12) nach Anspruch 1, wobei die Sonde (110) zur Übertragung des akustischen Vortex-Feldes mindestens eine piezoelektrische Stelle (101) oder mehrere Array-Elemente (mindestens vier Array-Elemente) piezoelektrischer Stellen (101) umfasst.

4. Vortex-Katheter-Thrombolyse-System (12) nach Anspruch 3, wobei der Krümmungsradius der piezoelektrischen Stelle (101) im Bereich von 1 mm bis 300 mm liegt.

5. Vortex-Katheter-Thrombolyse-System (12) nach Anspruch 1, wobei:
das Vortex-Katheter-Thrombolyse-System (12) ferner eine weitere radiale Sonde (110) zur Übertragung eines akustischen Vortex-Feldes umfasst;
der zweite innere Kanal (220) ferner mit einer weiteren piezoelektrischen Stelle versehen ist, wobei die beiden piezoelektrischen Stellen (101) an den beiden radialen Sonden (110) jeweils zur Vortex-Erzeugung verwendet werden, die beiden radialen Sonden (110) zur Übertragung des akustischen Vortex-Feldes so konfiguriert sind, dass sie eine Vortex-Bewegung an zwei Seiten ausführen, einer der piezoelektrischen Stellen (101) umfasst vier Kanäle, die in radialer Richtung des Katheters (200) angeordnet sind, die andere piezoelektrische Stelle (101) umfasst vier Kanäle, die in einer anderen radialen Richtung des Katheters (200) angeordnet sind, wobei eine Phasenverschiebung zwischen zwei beliebigen benachbarten Kanälen jeder piezoelektrischen Stelle (101) verwendet wird, um einen akustischen Vortex (300) in der entsprechenden radialen Richtung des Katheters (200) durch den Ultraschallwandler (105) zu erzeugen;
jede piezoelektrische Stelle (101) im Katheter (200) eingeschlossen ist und nicht aus dem Katheter (200) herausragt;
jeder Abgabeausgang, an dem die Medikamente abgegeben werden, in der Nähe des durch die piezoelektrische Stelle (101) erzeugten akustischen Vortex (300) ist, wodurch die Medikamente abgegeben und auf ein Zentrum des akustischen Vortex (300) in der entsprechenden radialen Richtung des Katheters (200) fokussiert werden.

## Revendications

1. Système de thrombolyse par cathéter à vortex (12), comprenant un transducteur ultrasonique (105), une sonde radiale (110) pour transmettre un champ acoustique en vortex, et un cathéter (200), dans lequel :
le cathéter (200) est connecté au transducteur ultrasonique (105) et est pourvu d'un premier canal interne (210) et d'un deuxième canal interne (220), le premier canal interne (210) étant configuré pour administrer des médicaments, le deuxième canal interne (220) étant pourvu d'une pastille piézoélectrique (101) sur la sonde radiale (110) configurée pour l'entraînement de vortex, et la pastille piézoélectrique (101) comprenant quatre canaux, **caractérisé en ce que** :
les quatre canaux sont disposés dans une direction radiale du cathéter (200), une différence de phase entre deux canaux adjacents quelconques de la pastille piézoélectrique (101) étant configurée pour générer un vortex acoustique (300) dans la direction radiale du cathéter (200) par le transducteur ultrasonique (105) ;
la pastille piézoélectrique (101) est enveloppée dans le cathéter (200) et n'est pas exposée à l'extérieur du cathéter (200) ; et
une sortie de délivrance où les médicaments sont administrés est adjacente au vortex acoustique (300) généré par la pastille piézoélectrique (101), de sorte que les médicaments sont délivrés et focalisés sur un centre du vortex acoustique (300) dans la direction radiale du cathéter (200).

2. Système de thrombolyse par cathéter à vortex (12) selon la revendication 1, dans lequel la sonde (110) pour transmettre le champ acoustique en vortex est connectée au cathéter (200) selon un mode d'adsorption magnétique, un mode de verrouillage ou un mode de collage.

3. Système de thrombolyse par cathéter à vortex (12) selon la revendication 1, dans lequel la sonde (110) pour transmettre le champ acoustique en vortex comprend au moins une pastille piézoélectrique (101) ou comprend plusieurs éléments en réseau (au moins quatre éléments en réseau) de pastilles piézoélectriques (101).

4. Système de thrombolyse par cathéter à vortex (12) selon la revendication 3, dans lequel le rayon de courbure de la pastille piézoélectrique (101) varie de 1 mm à 300 mm.

5. Système de thrombolyse par cathéter à vortex (12) selon la revendication 1, dans lequel :
le système de thrombolyse par cathéter à vortex (12) comprend en outre une autre sonde radiale (110) pour transmettre un champ acoustique en vortex ;
le deuxième canal interne (220) est en outre pourvu d'une autre pastille piézoélectrique, les deux pastilles piézoélectriques (101) sur les deux sondes radiales (110) étant respectivement utilisées pour l'entraînement de vortex, les deux sondes radiales (110) pour transmettre le champ acoustique en vortex étant configurées pour effectuer un mouvement de vortex sur deux côtés, l'une des pastilles piézoélectriques (101) comprenant quatre canaux disposés dans une direction radiale du cathéter (200), l'autre pastille piézoélectrique (101) comprenant quatre canaux disposés dans une autre direction radiale du cathéter (200), une différence de phase entre deux canaux adjacents quelconques de chaque pastille piézoélectrique (101) étant utilisée pour générer un vortex acoustique (300) dans la direction radiale correspondante du cathéter (200) par le transducteur ultrasonique (105) ;
chaque pastille piézoélectrique (101) est enveloppée dans le cathéter (200) et n'est pas exposée à l'extérieur du cathéter (200) ;
chaque sortie de délivrance où les médicaments sont administrés est adjacente au vortex acoustique (300) généré par la pastille piézoélectrique (101), de sorte que les médicaments sont délivrés et focalisés sur un centre du vortex acoustique (300) dans la direction radiale correspondante du cathéter (200).
